(19) Europäisches Patentamt
European Patent Office
Office européen des brevets

(11) **EP 0 867 177 B1**

(12) **EUROPÄISCHE PATENTSCHRIFT**

(45) Veröffentlichungstag und Bekanntmachung des Hinweises auf die Patenterteilung:
**08.10.2003   Patentblatt 2003/41**

(51) Int Cl.[7]: **A61K 9/14**, A61K 47/02,
A61K 31/07, A61K 31/355,
A61K 31/59, A61K 31/12,
A61K 31/015, A61K 31/20

(21) Anmeldenummer: **98102962.2**

(22) Anmeldetag: **20.02.1998**

(54) **Fliessfähige getrocknete Partikel**

Free-flowing dried particles

Particules séchées à écoulement libre

(84) Benannte Vertragsstaaten:
**BE CH DE DK ES FR GB IE IT LI NL SE**

(30) Priorität: **28.02.1997   CH 48297**

(43) Veröffentlichungstag der Anmeldung:
**30.09.1998   Patentblatt 1998/40**

(73) Patentinhaber: **F. HOFFMANN-LA ROCHE AG**
**4070 Basel (CH)**

(72) Erfinder:
• **Tritsch, Jean-Claude**
**68300 Saint-Louis (FR)**

• **Ulm, Johann**
**4104 Oberwil (CH)**

(74) Vertreter: **Kellenberger, Marcus, Dr. et al**
**F.Hoffmann-La Roche AG**
**Patent Department (PLP),**
**124 Grenzacherstrasse**
**4070 Basel (CH)**

(56) Entgegenhaltungen:
| | |
|---|---|
| **EP-A- 0 074 050** | **US-A- 2 858 215** |
| **US-A- 3 247 064** | **US-A- 3 445 563** |
| **US-A- 4 603 143** | **US-A- 4 670 247** |
| **US-A- 4 780 309** | **US-A- 4 906 478** |

**Beschreibung**

Die Erfindung betrifft fliessfähige getrocknete Partikel bestehend aus mindestens einer sich in einer Matrix aus mindestens einem Trägerstoff befindlichen oleophilen Substanz als Wirkstoff und einer Umhüllung.

Fliessfähige getrocknete Partikel bestehend aus mindestens einer sich in einer Matrix aus mindestens einem Trägerstoff befindlichen oleophilen Substanz als Wirkstoff und einer Umhüllung sind bekannt. Ihr Anteil an oleophiler Substanz als Wirkstoff beträgt höchstens 50 Gew. %.

Ein Verfahren zur Herstellung derartiger Partikel ist beispielsweise in der deutschen Patentschrift 10 35 319 bzw. im entsprechenden US-Patent 2756 177 beschrieben, bei dem eine Dispersion eines öligen Vitamins als Wirkstoff in einen grossen Ueberschuss eines Stärkepulvers mit einem Wassergehalt unter 8 % versprüht wird, wobei das trockene Stärkepulver die Sprühpartikel auffängt und den Sprühpartikeln soviel Wasser entzieht, dass diese bei gleichzeitiger Umhüllung mit Stärkepulver erstarren. Ein grosser Nachteil dieser Partikel besteht darin, dass ihnen etwa 15 % der Stärkemenge an der Oberfläche anhaften und somit die Partikel nur eine relativ geringe Wirkstoffmenge enthalten.

Nach einem analogen, in der US Patentschrift 3 445 563 beschriebenen Verfahren wird die Stärke durch ein Gemisch wasserabsorbierender und wassernichtabsorbierender anorganischer Substanzen ersetzt, um die Explosionsgefahr, die von der feinteiligen Stärke ausgeht, auszuschalten. Für optimale Ergebnisse ist ein 20-facher Ueberschuss des Auffangpulvers notwendig. Als wasserabsorbierende Komponente des Auffangpulvers wird unter anderem Calciumsilikat, insbesondere Calciumaluminiumsilikat, als öllösliche Wirkstoffe Vitamin A und Vitamin D genannt. Bei einer zwischen 245 000 und 532 000 I.E./g liegenden Wirkstoffaktivität enthalten die erhaltenen Partikel bis zu 19 % Auffangpulver als Umhüllung.

Ferner ist durch die europäische Patentanmeldung 0074050 ein Verfahren bekannt geworden, das zur Herstellung von trockenen, freifliessenden Pulvern leicht oxidierbarer Stoffe, wie der Vitamine oder Carotinoide, die von einem Kolloid eingehüllt sind, dient. Das Verfahren ist gekennzeichnet durch Dispergieren dieser Stoffe in einer wässrigen Lösung eines filmbildenden Kolloids, wobei das Kolloid die homogene Phase darstellt. Unter Zusatz eines oder mehrerer Stoffe aus der Gruppe der Mono-, Di- oder Polysaccharide wird die Dispersion in einem Sprühturm unter Mitverwendung eines Sprühhilfsmittels gesprüht und die versprühten Teilchen in einem Fliessbett aufgefangen. Dabei wird als Sprühhilfsmittel eine hydrophobe Kieselsäure oder ein Metallsalz einer höheren Fettsäure oder Gemische mit Kieselsäure, in der 0,02- bis 0,15-fachen Gewichtsmenge, bezogen auf die Dispersion oberhalb des Fliessbetts unter gleichmässiger Verteilung in den Sprühraum bei Temperaturen einführt, bei denen eine Erstarrung des Kolloids der versprühten Teilchen noch nicht eintritt. Die mit Hilfsmittel beladenen Teilchen, deren Kolloidmasse im wesentlichen nicht geliert ist, wird in einem Fliessbett aufgefangen und die Teilchen in an sich bekannter Weise im Fliessbett getrocknet.

Obwohl bei diesem Verfahren nur ein dünner, hydrophober Film des Sprühhilfsmittels erzeugt wird, der die während des Sprühens gebildeten Partikel soweit stabilisiert, dass ein Zusammenlaufen der Partikel bei Berührung im nicht erstarrtem Zustand verhindert wird, so dass die direkte Trocknung anschliessend auf einem Wirbelbett-Trockner möglich ist, hat das vorgeschlagene Verfahren den wesentlichen Nachteil, dass als Sprühhilfsmittel hydrophobe Kieselsäure zur Anwendung kommt. Der Einsatz von freier Kieselsäure in der weiterverarbeitenden pharmazeutischen oder Nahrungsmittelindustrie ist zumindest bedenklich, da sie die Gesundheit gefährdet, und daher in vielen Ländern für diese Zwecke nicht zugelassen.

Somit befriedigen die genannten Verfahren bzw. die danach hergestellten fliessfähigen getrockneten Partikel der eingangs genannten Art nicht, insbesondere nicht für den Einsatz in der Pharma- oder Nahrungsmittelindustrie.

Aufgabe der vorliegenden Erfindung ist es daher, die vorgenannten Nachteile zu überwinden. Insbesondere sollen fliessfähige getrocknete Partikel der eingangs genannten Art mit einem Wirkstoffanteil von 50 und mehr Gew. %, insbesondere von über 70 Gew. %, d.h. mit Wirkstoff hoch dosierte Partikel bereitgestellt und ein Verfahren zur Herstellung der Partikel angegeben werden. Das spezifische Ziel der Erfindung ist darüber hinaus, eine feste Verabreichungsform mit diesen fliessfähigen getrockneten Partikeln bereitzustellen, um einen möglichst hohen Wirkstoffanteil in die Verabreichungsform einzubringen.

Die Aufgabe wird erfindungsgemäss dadurch gelöst, dass fliessfähige getrocknete Partikel bereitgestellt werden, bestehend aus mindestens einer sich in einer Matrix aus mindestens einem Trägerstoff befindlichen oleophilen Substanz als Wirkstoff und einer Umhüllung, dadurch gekennzeichnet, dass die Umhüllung aus Calciumsilikat oder aus einer Mischung von Caiciumsilikat mit einer der folgenden Mischungskomponenten: mikrokristalline Cellulose, Magnesiumsilikat, Magnesiumoxid, Stearinsäure, Calciumstearat, Magnesiumstearat, hydrophile Kieselsäure, Kaolin und/oder Sterotex besteht, wobei der Anteil an Calciumsilikat zwischen 2 und 12 Gew.% beträgt, das Calciumsilikat ein $SiO_2/CaO$-Verhältnis von 1,65 - 2,65 aufweist, der Anteil an oleophiler Substanz 50 Gew. % oder mehr beträgt, und die mittlere Grösse der Partikel zwischen 80 µm und 1000 µm, insbesondere zwischen etwa 100 µm und etwa 800 µm liegt.

Bei einer Umhüllung, die nur aus Calciumsilikat besteht, kann der Anteil der oleophilen Substanz mehr als 70 Gew. % betragen, ohne dass vorerwähnte nachteilige Eigenschaften auch nur teilweise auftreten. Es konnten sogar erfindungsgemässe Partikel mit einem Anteil von 74,0 bis 78,0 Gew. % an oleophiler Substanz hergestellt werden.

Bei den erfindungsgemässen Partikeln, deren Umhüllung nur aus Calciumsilikat besteht, liegt der Anteil an Calciumsilikat zwischen 2 und 12 Gew. %, vorzugsweise etwa $\leq$ 7 Gew. %. In diesem im Vergleich zum Stand der Technik sehr geringen Anteil an umhüllender Substanz liegt der wesentliche Grund, dass sich ein sehr hoher Wirkstoffanteil, nämlich ein sehr hoher Anteil an olephiler Substanz, in die Matrix einarbeiten lässt, ohne dass die Handhabung der Partikel beeinträchtigt wird. Der geringe Anteil an umhüllender Substanz war insofern überraschend, da davon auszugehen ist, die sich chemisch inert verhaltenden, für diesen Zweck in Frage kommenden Substanzen würden sich alle in etwa der gleichen Menge an den Trägerstoff der Matrix heften.

Bei den erfindungsgemässen Partikeln, deren Umhüllung aus einer Mischung von Calciumsilikat mit einem oder mehreren der oben genannten Mischungskomponenten besteht, beträgt der Anteil an Calciumsilikat -Mischung zwischen 5 und 25 Gew. %.

Es stellte sich jedoch heraus, dass sowohl die chemische als auch physikalische Materialbeschaffenheit der Calciumsilikatpartikel von Bedeutung ist. So wurde überraschenderweise gefunden, dass Calciumsilikatpartikel besonders geeignet sind, wenn sie eine Grösse von $\leq$ 0,2 µm, insbesondere $\leq$ 0,1 µm, und eine spezifische Oberfläche von mindestens etwa 80 m$^2$/g bis etwa 180 m$^2$/g, vorzugsweise von etwa 95 m$^2$/g und 120 m$^2$/g, aufweisen und zu Aggregaten einer mittleren Grösse von etwa 5-20 µm, vorzugsweise 5-10 µm, agglomeriert sind. Das SiO$_2$/CaO-Verhältnis liegt zwischen 1,65 und 2,65.

Ferner ist es vorteilhaft, wenn das Calciumsilikat ganz oder teilweise in Form des Hydrats vorliegt. Das Calciumsilikat ist praktisch frei von kristalline Kieselsäure. Damit scheiden für die erfindungsgemässen Partikel zahlreiche auf dem Markt erhältliche Calciumsilikate als Umhüllungssubstanz aus, da diese mit kristalliner Kieselsäure versetzt sind.

Die oleophile Substanz ist mindestens eine aus der Gruppe: liphophile Vitamine oder deren Derivate, Carotinoide, insbesondere β-Carotin, und mehrfach ungesättigte Fettsäuren wie z.B. Arachidonsäure, Ecosapentoensäure, Docosahexaensäure, insbesondere mindestens ein Vitamin aus der Gruppe der Vitamine A, D, E und K oder ein Derivat davon, insbesondere Vitamin A-Acetat, Vitamin A-Palmitat und /oder Vitamin E-Acetat, vorzugsweise Vitamin E oder Vitamin E-Acetat.

Vitamin E beinhaltet synthetisch hergestellte Tocopherole oder eine Mischung natürlicher Tocopherole (mixed tocopherols).

Der Trägerstoff ist Cellulose, wasserlösliche Cellulosederivate, insbesondere Methylcellulose oder Hydroxypropylmethylcellulose, Maltodextrin, insbesondere Maltodextrin mit einem Dextroseäquivalentwert von etwa 18, ein Alginsäurederivat, insbesondere Natrium-, Calcium- oder Propylenglycolalginat, Calciumlactat, Gummi arabicum, Gelatine, insbesondere Fischgelatine, Zucker, Zuckeralkohol, Glycerin, modifizierte Stärke oder vorgelatinierte Getreidestärke, vorzugsweise Gelatine, insbesondere Fischgelatine. Gelatine mit einer Bloom-Zahl zwischen 0 und etwa 220 hat sich besonders bewährt. Der Trägerstoff oder die diesen enthaltende Matrix kann zusätzlich mindestens ein wasserlösliches Vitamin enthalten.

Die fliessfähigen getrockneten Partikel sind zur Herstellung von festen Verabreichungsformen, insbesondere in Form von Tabletten, gut geeignet, da sie ein hervorragendes Fliess- und Pressverhalten aufweisen, ohne dass über die übliche Menge hinaus Presshilfsmittel zugesetzt werden müssen.

Aufgrund der Möglichkeit, einen Wirkstoffanteil von 70 Gew % oder mehr (es wurden Anteile von 74 beziehungsweise 78 Gew. % ohne Probleme eingebracht) in die erfindungsgemässen Partikel einzubringen, eignen sich diese insbesondere zur Herstellung von Multivitamin-und Multimineraltabletten, da bei diesen ein möglichst hoher Wirkstoffanteil bei möglichst geringem Volumen beziehungsweise Gewicht angestrebt wird. Unter den Begriff "Multivitamin-und Multimineraltablette" sollen hier auch die Ausführungen als Brause- oder Kautablette verstanden werden.

Ein bevorzugtes Verfahren zur Herstellung der erfindungsgemässen Partikel ist dadurch gekennzeichnet, dass eine wässrige Emulsion aus der/den oleophilen Substanz/en und dem/den Trägerstoff/en nach dem Catchverfahren, insbesondere nach dem Powdercatchverfahren, in ein Catchmedium aus Calciumsilikat oder aus einer Mischung von Calciumsilikat mit einer oder mehreren Mischungskomponenten wie oben näher gekennzeichnet, gesprüht wird und die erhaltenen Partikel anschließend in an sich bekannter Weise getrocknet werden.

Feste Verabreichungsformen, insbesondere Multivitamintabletten, haben neben mindestens einer wasserlöslichen Substanz als Wirkstoff bevorzugt mindestens eine oleophile Substanz als Wirkstoff aus der Gruppe Carotinoide, insbesondere, β-Carotin, und Vitamine A, D, E und K oder ein Derivat davon. Der Vitamin E Anteil liegt vorzugsweise in einer Menge von 4 bis 50 Gew. % vor. Die Verabreichungsform kann neben den üblichen Hilfsstoffen zusätzlich mindestens ein Spurenelement, üblicherweise eingebracht in Form eines Minerals, und/oder mindestens ein wasserlösliches Vitamin enthalten.

Als Spurenelemente kommen insbesondere Mangan, Jod, Kalium, Magnesium, Calcium, Phosphor, Zink, Kupfer und Eisen zur Anwendung, wobei vorteilhafterweise Calcium zumindest zum Teil bereits durch die Umhüllung der erfindungsgemässen Partikel beigesteuert wird. Untergeordnet kommen noch Selen, Chrom, Chlor (als Chlorid), Molybdän, Nickel, Zinn, Silizium (gebunden), Vanadium und Bor zum Einsatz Silizium (gebunden) wird wie Calcium durch die erfindungsgemässen Partikel zumindest teilweise eingebracht. Mindestens eine Substanz aus der Gruppe Vitamin C, $B_1$, $B_2$, $B_6$, $B_{12}$, Pantothensäure, Calciumpantothenat, Folsäure, Biotin und Nicotinsäureamid wird für obgenannte Zwecke als wasserlösliche Substanz eingesetzt.

Weitere Einzelheiten und Vorteile ergeben sich aus den Beispielen.

Beispiele 1-3 beschreiben Partikel, die nur mit Calciumsilikat umhüllt sind.

Beipiel 4 betrifft die Umhüllung mit einer Mischung aus Calciumsilikat und mikrokristalliner Cellulose.

Beispiele 5-8 beschreiben Multivitamintabletten.

Beispiel 1

In einem 500 ml Gefäss wurden 38 g Fischgelatine getrocknet (Bloom- Zahl 0) vorgelegt. Dann wurden 95 ml entionisiertes Wasser zugesetzt und das Ganze unter Rühren mit einer Minzerscheibe mit 1000 Umdrehungen/ Minute (U/Min.) bei 40-50° C in Lösung gebracht, was die Matrix ergab. Hierauf wurden 154 g Tocopherol-Acetat in diese Matrix hineinemulgiert und 15 Minuten nachgerührt. Während des Emulgierens und Nachrührens betrug die Tourenzahl der Minzerscheibe 4800 U/Min. Nach dieser Zeit wies die innere Phase der Emulsion eine mittlere Teilchengrösse von etwa 250 nm auf. Die Emulsion wurde dann mit 130 ml entionisiertem Wasser verdünnt und auf 65° C erwärmt. In einer Laborsprühwanne wurden anschliessend 225 g Calciumsilikat (Micro-Cel E der Firma Celite Corp., USA mit einem $SiO_2$/CaO-Verhältnis von 1,65) vorgelegt und auf mindestens 0° C gekühlt. Die Emulsion wurde mittels einer rotierenden Sprühdüse in die Sprühwanne eingesprüht. Die so erhaltenen mit Calciumsilikat umhüllten Partikel wurden von dem überschüssigen Calciumsilikat abgesiebt (Siebfraktion 100-800 μm) und bei Raumtemperatur mittels eines Luftstroms getrocknet. Man erhielt 190 g mit Calciumsilikat umhüllte Partikel, die hervorragende Fliesseigenschaften aufwiesen, vollkommen trocken waren und damit eine sehrgute Handhabbarkeit aufwiesen. Der Calciumsilikatanteil betrug 7 Gew. %.

Beispiel 2

In einem zu Beispiel 1 analogen Versuch wurde die Fischgelatine durch eine Gelatine für pharmazeutische Zwecke mit einer Bloom-Zahl 220 der Firma Croda, England ersetzt. Die Ausbeute betrug 195 g. Das Produkt hatte die gleich guten anwendungstechnischen Eigenschaften wie das von Beispiel 1.

Beispiel 3

Das Beispiel 1 wurde analog wiederholt, jedoch unter Verwendung eines Calciumsilikats mit einem $SiO_2$/CaO-Verhältnis über 2,65. Es bildeten sich mit Calciumsilikat umhüllte Partikel, deren Tocopherol-Acetat-Anteil nur 44,3 Gew. % betrug. Der Gewichtsanteil an Calciumsilikat stieg auf über 7 Gew. %. Ein Calciumsilikat mit einem $SiO_2$/CaO-Verhältnis von über 2,65 ist daher ungeeignet.

Beispiel 4

In einem 500 ml Gefäss wurden 36 g high molecular Fischgelatine (Norland Products Incorp.) getrocknet (Bloom- Zahl 0) vorgelegt. Dann wurden 95 ml entionisiertes Wasser zugesetzt und das Ganze unter Rühren mit einer Minzerscheibe mit 1000 Umdrehungen/Minute (U/Min.) bei 40-50° C in Lösung gebracht, was die Matrix ergab. Hierauf wurden 156 g Tocopherol-Acetat in diese Matrix hineinemulgiert und 15 Minuten nachgerührt. Während des Emulgierens und Nachrührens betrug die Tourenzahl der Minzerscheibe 4800 U/Min. Nach dieser Zeit wies die innere Phase der Emulsion eine mittlere Teilchengrösse von etwa 300 nm auf. Die Emulsion wurde dann mit 135 ml entionisiertem Wasser verdünnt und auf 65° C erwärmt. In einer Laborsprühwanne wurden anschliessend 410g einer Mischung aus Calciumsilikat (Micro-Cel E der Firma Celite Corp., USA mit einem $SiO_2$/CaO-Verhältnis von 1,65) und mikrokristalliner Cellulose (VIVAPUR Type 105) vorgelegt. Das Verhältnis von VIVAPUR Type 105 zu Micro-Cel E betrug 5,66:1. Die Mischung wurde auf mindestens 0° C gekühlt. Die Emulsion wurde mittels einer rotierenden Sprühdüse in die Sprühwanne eingesprüht. Die so erhaltenen mit Calciumsilikat-Cellulose umhüllten Partikel wurden von der überschüssigen Calciumsilikat-Cellulose-Mischung abgesiebt (Siebfraktion 100-800 μm) und bei Raumtemperatur mittels eines Luftstroms getrocknet. Man erhielt 222.1 g mit Calciumsilikat-Cellulose umhüllte Partikel, die hervorragende Fliesseigenschaften aufwiesen, vollkommen trocken waren und damit eine seht gute Handhabbarkeit aufwiesen. Der Calciumsilikat-Cellulose-Anteil betrug 21Gew. %.

Beispiel 5

Es wurden Multivitamintabletten aus den folgenden Komponenten auf einer Tablettenmaschine Comprex II, Stempel 16 x 7,42 mm unter Drücken von 5 bis 50 KN hergestellt.

| | |
|---|---|
| Pulverförmiges Produkt aus Beispiel 1 | 147,0 mg |
| Beta-Tab 7.5 (7,5 %ige tablettierbare β-Carotin-Form von ROCHE) | 96,0 mg |
| Ascorbinsäure 90 % Gr. | 244,5 mg |
| Avicel PH 102 | 130,0 mg |
| Lactose DCL 2 l | 50.0 mg |
| Gesamtgewicht der Tablette | 668,0 mg |

Die resultierende Tablettenhärte betrug 20 bis 140 N. Die Tabletten waren trocken.

Beispiel 6

Analog wie in Beispiel 5 wurde eine Multivitamintablette (E 75 % Formula, enthaltend 75 Gew. % Vitamin E-Partikel) unter Verwendung erfindungsgemässer Partikel mit einem Vitamin E -Gehalt von 75 Gew. % als Wirkstoff hergestellt und mit einer herkömmlichen Vitamintablette ( E 50 % Formula, enthaltend 50 Gew. % Vitamin E-Partikel) derselben Zusammensetzung verglichen.

| | E 75 % Formula | E 50 % Formula |
|---|---|---|
| Tablettengewicht | 808,3 mg | 948,3 mg |
| β-Carotin ∗ | 34,5 mg | 34,5 mg |
| Vitamin E | 280,0 mg | 420,0 mg |
| Vitamin C 90 | 291,7 mg | 291,7 mg |
| Avicel | 161,6 mg | 161,6 mg |
| Microcel C | 24,3 mg | 24,3 mg |
| PVP XL | 16,2 mg | 16,2 mg |

∗ Beta-Tab 20 (20 %ige tablettierbare β-Carotin-Form von ROCHE)

Hieraus ist ersichtlich, dass mit den erfindungsgemässen Partikeln Tabletten mit geringerem Gewicht (beziehungsweise entsprechend geringerem Volumen) hergestellt werden können als mit herkömmlich hergestellten, Vitamin E enthaltenden Partikeln.

Beispiel 7

Analog zu Beispiel 5 wurde eine Multivitamintablette (E 75 % Formula, enthaltend 75 Gew. % Vitamin E-Partikel) unter Verwendung erfindungsgemässer Partikel mit einem Vitamin E -Gehalt von 75 Gew. % als Wirkstoff hergestellt und mit einer herkömmlichen Vitamintablette ( E 50 % Formula, enthaltend 50 Gew. % Vitamin E-Partikel) derselben Zusammensetzung verglichen.

| | E 75 % Formula | E 50 % Formula |
|---|---|---|
| Vitamin/Mineralmischung | 1294.8 mg | 1294.8 mg |
| Vitamin E | 70.7 mg | 106.0 mg |
| Microcellulose | 57.2 mg | 0.0 mg |
| PVP XL | 6.6 mg | 30.0 mg |
| Stearinsäure | 2.0 mg | 2.0 mg |
| Magnesiumstearat | 4.2 mg | 4.2 mg |

Die resultierende Tablettenhärte betrug 210N. Mit E 75 % Formula konnte der Anteil an Zerfallsmittel PVP XL reduziert werden, ohne dass sich die Auflösezeit der Tablette (weniger als 5 Minuten) änderte.

Beispiel 8

Analog zu Beispiel 5 wurde eine Multivitamintablette (E 75 % Formula, enthaltend 75 Gew. % Vitamin E-Partikel) unter Verwendung erfindungsgemässer Partikel mit einem Vitamin E -Gehalt von 75 Gew. % als Wirkstoff hergestellt. Die Tablette enthielt kein Zerfallsmittel und hatte die folgende Zusammensetzung.

|  | E 75 % Formula |
|---|---|
| Vitamin/Mineralmischung | 1294.8 mg |
| Vitamin E | 70.7 mg |
| Microcellulose | 58.0 mg |
| Stärke | 15.0 mg |
| Stearinsäure | 2.0 mg |
| Magnesiumstearat | 4.2 mg |

Die resultierende Tablettenhärte betrug 210N. Die Auflösezeit der Tablette betrug weniger als 5 Minuten.

**Patentansprüche**

1.  Fliessfähige getrocknete Partikel, bestehend aus mindestens einer sich in einer Matrix aus mindestens einem Trägerstoff befindlichen oleophilen Substanz als Wirkstoff und einer Umhüllung, **dadurch gekennzeichnet, dass** die Umhüllung aus Calciumsilikat oder aus einer Mischung von Calciumsilikat mit einer der folgenden Mischungskomponenten: mikrokristalline Cellulose, Magnesiumsilikat, Magnesiumoxid, Stearinsäure, Calciumstearat, Magnesiumstearat, hydrophile Kieselsäure, Kaolin und/oder Sterotex besteht, wobei der Anteil an Calciumsilikat zwischen 2 und 12 Gew.% beträgt, das Calciumsilikat ein $SiO_2$/CaO-Verhältnis von 1,65 - 2,65 aufweist, der Anteil an oleophiler Substanz 50 Gew. % oder mehr beträgt, und die mittlere Grösse der Partikel zwischen etwa 80 $\mu$m und etwa 1000 $\mu$m, insbesondere zwischen etwa 100 $\mu$m und etwa 800 $\mu$m liegt.

2.  Fliessfähige getrocknete Partikel nach Anspruch 1, **dadurch gekennzeichnet, dass** die Umhüllung aus Calciumsilikat besteht.

3.  Fliessfähige getrocknete Partikel nach Anspruch 1 oder 2, **dadurch gekennzeichnet, dass** der Anteil der oleophilen Substanz 74,0 bis 78,0 Gew.% beträgt und die mittlere Grösse der Partikel zwischen 80 $\mu$m und 1000 $\mu$m, insbesondere zwischen 100 $\mu$m und 800 $\mu$m, liegt.

4.  Fliessfähige getrocknete Partikel nach Anspruch 2 oder 3, **dadurch gekennzeichnet, dass** der Anteil an Calciumsilikat $\leq$ 7 Gew.% beträgt.

5.  Fliessfähige getrocknete Partikel nach einem der Ansprüche 1 - 4, **dadurch gekennzeichnet, dass** das Calciumsilikat aus Partikeln der Grösse von $\leq$ 0,2 $\mu$m, insbesondere $\leq$ 0,1 $\mu$m besteht, die zu Aggregaten einer mittleren Grösse von etwa 5 - 20 $\mu$m, insbesondere 5 - 10 $\mu$m agglomeriert sind und eine spezifische Oberfläche von mindestens 80 $m^2$/g bis etwa 180 $m^2$/g, vorzugsweise zwischen 95 $m^2$/g und 120 $m^2$/g, aufweisen; und gegebenenfalls ganz oder teilweise ein Calciumsilikathydrat ist.

6.  Fliessfähige getrocknete Partikel nach mindestens einem der Ansprüche 1 - 5, **dadurch gekennzeichnet, dass** die oleophile Substanz mindestens eine Substanz aus der Gruppe der lipophilen Vitamine oder deren Derivate, vorzugsweise aus der Gruppe der Vitamine A, D, E und K oder deren Derivate, ganz besonders bevorzugt Vitamin A-Acetat, Vitamin A-Palmitat, Vitamin E und/oder Vitamin E-Acetat; Carotinoide, insbesondere $\beta$-Carotin; und/oder eine Substanz aus der Gruppe mehrfach ungesättigter Fettsäuren; ist.

7.  Fliessfähige getrocknete Partikel nach mindestens einem der Ansprüche 1 - 6, **dadurch gekennzeichnet, dass** der Trägerstoff mindestens eine Stoff aus der Gruppe Cellulose, wasserlösliche Cellulosederivate, insbesondere Methylcellulose oder Hydroxypropylmethylcellulose, Maltodextrin, Maltodextrin, insbesondere Maltodextrin mit einem Dextroseäquivalentwert von etwa 18; Alginsäurederivat, insbesondere Natrium-, Calcium- oder Propylenglycolalginat; Calciumlactat; Gummi arabicum; Gelatine, insbesondere Fischgelatine, Zucker; Zuckeralkohol; Glycerin; modifizierte Stärke und vorgelatinierte Getreidestärke ist.

**8.** Fliessfähige getrocknete Partikel nach mindestens einem der Ansprüche 1 - 7, **dadurch gekennzeichnet, dass** in der Matrix zusätzlich mindestens ein wasserlösliches Vitamin enthalten ist.

**9.** Verfahren zur Herstellung der fliessfähigen getrockneten Partikel nach mindestens einem der Ansprüche 1 - 8, **dadurch gekennzeichnet, dass** eine wässrige Emulsion aus der/den oleophilen Substanz/en und dem/den Trägerstoff/en nach dem Catchverfahren, insbesondere nach dem Powdercatchverfahren, in ein Catchmedium aus Calciumsilikat oder aus einer Mischung von Calciumsilikat mit einer oder mehreren Mischungskomponenten, insbesondere in ein Catchmedium aus Calciumsilikat gesprüht wird und die erhaltenen Partikel anschliessend in an sich bekannter Weise getrocknet werden.

**10.** Feste Verabreichungsform, insbesondere Multivitamintablette, hergestellt aus fliessfähigen getrockneten Partikeln gemäss mindestens einem der Ansprüche 1 - 8.

**11.** Feste Verabreichungsform, insbesondere Multivitamintablette, nach Anspruch 10, **dadurch gekennzeichnet, dass** der Anteil an Vitamin E in der Tablette zwischen etwa 4 und 50 Gew. % beträgt.

**12.** Verwendung der fliessfähigen getrockneten Partikel nach mindestens einem der Ansprüche 1 - 8 zur Herstellung einer festen Verabreichungsform, insbesondere in Form einer Tablette; ganz besonders bevorzugt zur Herstellung einer Multivitamintablette, die gegebenenfalls zusätzlich mindestens ein Spurenelement, üblicherweise eingebracht in Form eines Minerals, enthält.

**13.** Verwendung der fliessfähigen getrockneten Partikel nach mindestens einem der Ansprüche 1-8 in der pharmazeutischen oder Nahrungsmittelindustrie.

**Claims**

**1.** Flowable dry particles consisting of at least one oleophilic substance as the active ingredient present in a matrix of at least one carrier material and a coating, **characterized in that** the coating consists of calcium silicate or of a mixture of calcium silicate with one of the following mixture components: microcrystalline cellulose, magnesium silicate, magnesium oxide, stearic acid, calcium stearate, magnesium stearate, hydrophilic silicic acid, kaolin and/ or Sterotex, with the calcium silicate content being between 2 and 12 wt.%, the calcium silicate having a $SiO_2/CaO$ ratio of 1.65-2.65, the content of oleophilic substance being 50 wt.% or above and the average particle size lying between 80 μm and 1000 μm, especially between 100 μm and 800 μm.

**2.** Flowable dry particles according to claim 1, **characterized in that** the coating consists of calcium silicate.

**3.** Flowable dry particles according to claim 1 or 2, **characterized in that** the content of oleophilic substance is 74.0 to 78.0 wt.% and the average particle size lies between 80 μm and 1000 μm, especially between 100 μm and 800 μm.

**4.** Flowable dry particles according to claim 2 or 3, **characterized in that** the calcium silicate content is ≤ 7 wt.%.

**5.** Flowable dry particles according to one of claims 1-4, **characterized in that** the calcium silicate consists of particles with a size of ≤ 0.2 μm, especially ≤ 0.1 μm, which are agglomerated to aggregates with an average size of about 5-20 μm, especially 5-10 μm, and which have a specific surface area of at least 80 $m^2$/g to 180 $m^2$/g, preferably between 95 $m^2$/g and 120 $m^2$/g,; and is optionally wholly or partially a calcium silicate hydrate.

**6.** Flowable dry particles according to at least one of claims 1-5, **characterized in that** the oleophilic substance is at least one substance from the group of lipophilic vitamins or their derivatives, preferably from the group of vitamins A, D, E and K or their derivatives, particularly vitamin A acetate, vitamin A palmitate, vitamin E and/or vitamin E acetate; carotinoids, especially β-carotene; and/or a substance from the group of polyunsaturated fatty acids.

**7.** Flowable dry particles according to at least one of claims 1-5, **characterized in that** the carrier material is at least one substance from the group of cellulose, water-soluble cellulose derivatives, especially methyl cellulose or hydroxypropylmethyl cellulose, maltodextrin, maltodextrin, especially maltodextrin with a dextrose equivalent of about 18; alginic acid derivative, especially sodium, calcium or propylene glycol alginate; calcium lactate; gum arabic; gelatin, especially fish gelatin, sugar; sugar alcohol; glycerol; modified starch and pre-gelatinized cereal starch.

8. Flowable dry particles according to at least one of claims 1-7, **characterized in that** at least one water-soluble vitamin is additionally present in the matrix.

9. A process for the manufacture of the flowable dry particles according to at least one of claims 1-8, **characterized by** spraying an aqueous emulsion of the oleophilic substance(s) and the carrier material(s) according to the catch process, especially according to the powder catch process, into a catch medium of calcium silicate or of a mixture of calcium silicate with one or more mixture components, especially into a catch medium of calcium silicate, and subsequently drying the particles in a manner known per se.

10. A solid administration form, especially multivitamin tablets, produced from flowable dry particles in accordance with one of claims 1-8.

11. A solid administration form, especially multivitamin tablets, according to claim 10, **characterized in that** the content of vitamin E in the tablets is between 4 and 50 wt.%.

12. The use of the flowable dry particles according to at least one of claims 1-8 for the production of a solid administration form, especially in the form of a tablet; particularly for the production of a multivitamin tablet which optionally additionally contains at least one trace element, usually incorporated in the form of a mineral.

13. The use of the flowable dry particles according to at least one of claims 1-8 in the pharmaceutical or food industry.


## Revendications

1. Particules séchées à écoulement libre, comprenant unc substance oléophile au moins comme principe actif, se trouvant dans une matrice constituée d'un support au moins, et une enveloppe, **caractérisées en ce que** l'enveloppe est constituée de silicate de calcium ou d'un mélange de silicate de calcium et d'un des composants de mélange suivants : cellulose microcristalline, silicate de magnésium, oxyde de magnésium, acide stéarique, stéarate de calcium, stéarate de magnésium, acide silique hydrophile, Kaolin et/ou Sterotcx, le pourcentage de silicate de calcium se trouvant entre 2 et 12 % en poids, le rapport de silicate de calcium $SiO_2/CaO$ étant de 1,65 à 2,65, le pourcentage de substance oléophile étant de 50 % en poids ou plus, et la dimension moyenne des particules étant située entre 80 $\mu$m environ et 1000 $\mu$m environ, notamment entre 100 $\mu$m et 800 $\mu$m.

2. Particules séchées à écoulement libre selon la revendication 1, **caractérisées en ce que** l'enveloppe est en silicate de calcium.

3. Particules séchées à écoulement libre selon la revendication 1 ou 2, **caractérisées en ce que** le pourcentage de substance oléophile est de 74,0 à 78,0 % en poids, et que la dimension moyenne des particules est située entre 80 $\mu$m et 1000 $\mu$m, notamment entre 100 $\mu$m et 800 $\mu$m.

4. Particules séchées à écoulement libre selon la revendication 2 ou 3, **caractérisées en ce que** le pourcentage de silicate de calcium est de $\leq$ 7 % en poids.

5. Particules séchées à écoulement libre selon l'une quelconque des revendications 1 à 4, **caractérisées en ce que** le silicate de calcium consiste en des particules dont la dimension est de $\leq$ 0,2 $\mu$m, notamment de $\leq$ 0,1 $\mu$m, agglomérées en agrégats de dimension moyenne de 5 à 20 $\mu$m environ, notamment 5 à 10 $\mu$m, comportant une surface spécifique de 80 $m^2/g$ au moins à 180 $m^2/g$, notamment située entre 95 $m^2/g$ et 120 $m^2/g$; le silicate de calcium étant le cas échéant un hydrate de silicate de calcium.

6. Particules séchées à écoulement libre selon l'une quelconque des revendications 1 à 5 au moins, **caractérisées en ce que** la substance oléophile est une substance au moins du groupe des vitamines lipophiles ou de ses dérivés, avantageusement du groupe des vitamines A, D, E et K ou de ses dérivés, et particulièrement avantageusement de la vitamine A-acétate, vitamine A-parmitate, vitamine E et/ou vitamine E-acétate; des carotinoïdes, notamment de la carotine $\beta$ ; et/ou une substance du groupe des acides gras non saturés multiples.

7. Particules séchées à écoulement libre selon l'une quelconque des revendications 1 à 6 au moins, **caractérisées en ce que** le support est une substance au moins du groupe cellulose, dérivés de cellulose solubles dans l'eau, notamment la méthylcellulose ou hydroxypropylméthylcellulose, maltodextrine, notamemment maltodextrine avec valeur équivalente en dextrose de 18 environ ; dérivé d'acide alginique, notamment l'alginate de sodium, l'alginate de calcium ou le glycoloalginate propylénique; lactate de calcium; gomme arabique ; gélatine, notamment la gélatine de poisson, sucre ; alcool de sucre, glycérine ; amidon gonflant et amidon de céréales prégélatinisé.

8. Particules séchées à écoulement libre selon l'une quelconque des revendications 1 à 7 au moins, **caractérisées en ce que** la matrice comprend en plus une vitamine au moins soluble dans l'eau.

9. Procédé destiné à la fabrication des particules séchées à écoulement libre selon l'une quelconque des revendications 1 à 8 au moins, **caractérisé en ce qu'**une émulsion aqueuse obtenuc à partir de la (des) substance(s) oléophile(s) et du (des) support(s) est pulvérisée selon le procédé Catch, notamment selon le procédé Powdercatch dans un médium catch de silicate de calcium ou d'un mélange de silicate de calcium avec un ou plusieurs composants de mélange, notamment dans un médium catch de silicate de calcium, les particules ainsi obtenues étant cnsuite séchées selon les méthodes traditionnelles.

10. Mode d'administration solide, notamment un comprimé multivitamine, fabriqué à base de particules séchées à écoulement libre selon l'une des revendications 1 à 8 au moins.

11. Mode d'administration solide, notamment un comprimé multivitamine selon la revendication 10, **caractérisé en ce que** le pourcentage de vitamine E dans le comprimé est situé entre 4 et 50% en poids.

12. Utilisation des particules séchées à écoulement libre selon l'une quelconque des revendication 1 à 8 au moins en vue de la fabrication d'un mode d'administration solide, notamment sous forme de comprimé ; avantageusement mis en jeu pour la fabrication d'un comprimé multivitamine qui, le cas échéant, comprend un oligoélément, habituellement incorporé sous forme d'un minéral.

13. Utilisation des particules séchées à écoulement libre selon l'une quelconque des revendication 1 à 8 au moins dans l'industrie pharmaceutique ou agro-alimentaire.